# EUROPEAN PATENT APPLICATION

(11) **EP 4 215 612 A1**
(43) Date of publication of application: **26.07.2023**
(21) Application number: 22152438.2
(22) Date of filing: 20.01.2022
(51) Int. Cl.: C12N 15/10, C12N 9/22

(54) **GENOME EDITING WITH CRISPR/CAS NUCLEASES COMPRISING COLLATERAL ACTIVITY**

(71) Applicant: Sartorius Stedim Cellca GmbH, 89081 Ulm (DE); BRAIN Biotech AG, 64673 Zwingenberg (DE)
(72) Inventor: The designation of the inventor has not yet been filed
(74) Representative: Wohlfahrt, Jan Günther

(57) **Abstract**

The present invention refers to a method for editing the genomic sequence of at least one cell at at least one specific target site with an endonuclease with ability of collateral cleavage of RNA and/or DNA, a kit comprising an endonuclease to perform the method according to the present invention and the use of an endonuclease with ability of collateral cleavage of RNA and/or DNA for editing the genomic sequence of at least one cell at a specific sequence site.

## Description

The present invention refers to a method for editing the genomic sequence of at least one cell at at least one specific target site with an endonuclease with ability of collateral cleavage of RNA and/or DNA, a kit comprising an endonuclease to perform the method according to the present invention and the use of an endonuclease with ability of collateral cleavage of RNA and/or DNA for editing the genomic sequence of at least one cell at a specific sequence site.

Genome editing using classical CRISPR/Cas nucleases such as Cas9 or Cpf1 is a well-established and straightforward process. Nevertheless, there are a number of problems and disadvantages related to this technology:
- Undesired editing events at non-target sites can occur, so called off-target effects. These can result in cells with unwanted modifications and phenotypes or compromised performance.
- For most genome edits, editing efficiencies are clearly below 90%, resulting in a significant percentage of unedited or wrongly edited cells. Especially in case of more complicated edits, such as gene knock-ins or challenging knock-outs, editing efficiencies are typically even further reduced, often leading to more than 80-90 % not/wrongly edited cells.
- For most genome editing targets, the direct selection of edited cells based on phenotypes is very complex or even impossible. This necessitates the generation and genotypic screening of clones in order to isolate and identify correctly edited cells. Especially in case of low editing efficiencies, high numbers of clones have to be analyzed.

Shortcomings and disadvantages of state of the art technologies for genome editing are therefore the occurrence of undesired off-target edits and/or the occurrence of unedited and wrongly edited cells and/or the missing or limited possibility to directly select for correctly edited cells based on their phenotype. In order to fully circumvent these drawbacks and obtain cells that are correctly edited and do/do not contain off-target edits, extensive clone characterization and screening are required. This can result in significantly increased workloads, costs and timelines.

The described issues can have negative impacts on or even prohibit the application of edited cells in experimental or commercial set ups. Furthermore, they can clearly increase workloads, project costs and timelines, because extensive clone characterization and screening are required.

The technical problem underlying the present invention is to overcome the outlined disadvantages of the state of the art. The technical problem is specially to provide a method which allows for genome editing without off-target effects as well as for the automatic removal of unedited and wrongly edited cells, resulting in pure populations with target edited cells.

The present invention solves the underlying technical problem by the subject matter of the independent claims.

The present invention solves the underlying technical problem preferably by a method for editing the genomic sequence of at least one cell at at least one specific target site comprising the steps:
a) introducing into the at least one cell an endonuclease with ability of collateral cleavage of RNA (CA nuclease) and/or DNA or a DNA or RNA with a sequence encoding such an endonuclease, at least one donor DNA comprising the at least one edited sequence of the at least one specific target site and at least one guide RNA (gRNA) or a DNA coding for such a gRNA, and
b) cultivating the at least one cell,
wherein the at least one guide RNA comprises a sequence binding to the genomic sequence of the at least one target site,
wherein the at least one donor DNA comprises a first homology arm located upstream of the edited sequence and a second homology arm located downstream of the edited sequence, wherein the sequence of the first homology arm is at least 80 % identical to the genomic sequence upstream of the at least one target site and the sequence of the second homology arm is at least 80 % identical to the genomic sequence downstream of the at least one target site.

The inventors have found that advantageously endonucleases with ability of collateral cleavage of RNA and/or DNA can be used in methods for editing the genomic sequence of at least one cell at at least one specific target site. For example, certain CRISPR/Cas systems comprise nucleases with so called "collateral activity" (CA nucleases), which means the nonspecific degradation of RNA or DNA upon target recognition by the nuclease. For example, the most prominent example of CRISPR CA nucleases acting on RNA level is Cas13a, whereas so far, the only known CA nuclease acting on DNA level is BEC, a chimeric enzyme.

The mode of action of CA nucleases can be described in the following:
1) A so-called guide RNA (gRNA) that is characterized by a sequence complementary to a sequence stretch of the targeted RNA/genomic DNA is designed.
2) The CA nuclease and the specific gRNA are transferred to or produced in the cell.
3) The gRNA binds to the CA nuclease and guides it to the target sequence on the RNA/genomic DNA.
4) Target recognition triggers the collateral activity of the nuclease resulting in the nonspecific degradation of RNA/genomic DNA.
5) The cell dies due to the extensive, universal degradation of RNA/DNA.

This mode of action can be used advantageously in genome editing according to the method of the present invention, that differs from approaches based on classical CRISPR Cas nucleases. In contrast to current technologies, CA nuclease-based gene editing is free from off-target effects and allows the automatic deletion of unedited or wrongly edited cells, resulting in pure cell populations consisting of target edited cells. The present invention allows therefore for the easy and efficient generation and selection of correctly edited, off-target free cells without the need to increase workloads, costs or timelines.

In a preferred embodiment of the invention, a culture of one cell or of at least 100 cells, more preferably of at least 10000 cells, even more preferably 1000000 cells is used. The method can be used accordingly for single cell cloning or for cloning of a population of cells.

In a preferred embodiment of the invention the at least one cell or the cells of the cell culture are mammalian cells. In an alternative embodiment of the invention the at least one cell or the cells of the cell culture are plant cells, yeast cells or bacterial cells.

In a preferred embodiment of the invention the at least one cell or the cells of the cell culture are cells of a cell line, preferably cells of a mammalian cell line. In a preferred embodiment of the invention the at least one cell or the cells of the cell culture are CHO cells.

In a preferred embodiment of the invention the method comprises the further step c) isolating the living cells.

In a preferred embodiment of the invention the endonuclease or a nucleotide with a sequence encoding the endonuclease, the at least one donor DNA comprising the edited sequence and the at least one guide RNA are introduced into the at least one cell via transfection. The skilled person knows suitable transfection systems and methods in the state of the art.

There are a number of options, in which form the individual components can be delivered to the cells: The donor DNA can be provided as plasmid, as linear dsDNA or as linear ssDNA. The gRNA can be provided as plasmid or as synthetic RNA, The CA nuclease can be provided as plasmid, as mRNA or as protein. The different form of delivering the donor DNA, gRNA and CA nuclease to the cells can be combined.

Also, there are a number of options for the delivery modes of the donor DNA, the gRNA and the CA nuclease. The delivery, preferably transfection, can be made in a single step, in two steps or in three steps.

In a single step delivery, preferably transfection, all three, the donor DNA, the gRNA and the CA nuclease are delivered together. In a twostep delivery, preferably transfection, preferably in a first step the donor DNA is delivered separately and in a second step the gRNA and the CA nuclease are delivered together. In a three-step delivery, preferably transfection, preferably in a first step the donor DNA is delivered separately in a second step the gRNA is delivered separately and in a third step the CA nuclease is delivered separately. Of course, also other combinations are possible.

In a preferred embodiment of the invention the endonuclease or a DNA or RNA with a sequence encoding the endonuclease, the at least one donor DNA comprising the edited sequence and the at least one guide RNA or a DNA coding for such a gRNA are introduced into the at least one cell together in one transfection step or in sequential separate steps in any order and combination.

In a preferred embodiment of the invention, in step a1) the at least one donor DNA is introduced into the at least one cell as linear ssDNA, wherein preferably in following step a2) the at least one guide RNA is introduced into the at least one cell as synthetic RNA together with the endonuclease protein.

In a preferred embodiment, the donor DNA is introduced as circular or linearized plasmid. In an alternative embodiment, the donor DNA is introduced as linear dsDNA or linear ssDNA.

In a preferred embodiment of the invention the endonuclease is a CRISPR-associated endonuclease.

In a preferred embodiment of the invention the endonuclease comprises collateral RNA cleavage activity, preferably is a type III or VI Cas nuclease, more preferably a type VI Cas nuclease, more preferably a Cas13 nuclease, most preferably a Cas13a nuclease.

Preferably a Cas13a nuclease is used as an endonuclease comprising collateral RNA cleavage activity

In a preferred embodiment of the invention the endonuclease comprises collateral DNA cleavage activity, preferably is a BEC nuclease. The BEC nuclease is described in PCT/EP2021/000081.

In a preferred embodiment, upon genomic integration of the edited sequence at the at least one target site via homologous recombination, the sequence of the at least one target site is changed in such a way that the at least one guide RNA sequence cannot bind to the modified at least one target site any more. In a preferred embodiment, upon genomic integration of the edited sequence at the at least one target site via homologous recombination, the sequence of the at least one target site is changed in such a way that that the collateral cleavage of the endonuclease gets activated.

In a preferred embodiment the sequence of the first homology arm is at least 81 % identical to the genomic sequence upstream of the at least one target site and the sequence of the second homology arm is at least 81 % identical to the genomic sequence downstream of the at least one target site. In a preferred embodiment the sequence of the first homology arm is at least 85 % identical to the genomic sequence upstream of the at least one target site and the sequence of the second homology arm is at least 85 % identical to the genomic sequence downstream of the at least one target site. In a preferred embodiment the sequence of the first homology arm is at least 90 % identical to the genomic sequence upstream of the at least one target site and the sequence of the second homology arm is at least 90 % identical to the genomic sequence downstream of the at least one target site. In a preferred embodiment the sequence of the first homology arm is at least 91 % identical to the genomic sequence upstream of the at least one target site and the sequence of the second homology arm is at least 91 % identical to the genomic sequence downstream of the at least one target site. In a preferred embodiment the sequence of the first homology arm is at least 95 % identical to the genomic sequence upstream of the at least one target site and the sequence of the second homology arm is at least 95 % identical to the genomic sequence downstream of the at least one target site. In a preferred embodiment the sequence of the first homology arm is at least 97 % identical to the genomic sequence upstream of the at least one target site and the sequence of the second homology arm is at least 97 % identical to the genomic sequence downstream of the at least one target site. In a preferred embodiment the sequence of the first homology arm is at least 98 % identical to the genomic sequence upstream of the at least one target site and the sequence of the second homology arm is at least 98 % identical to the genomic sequence downstream of the at least one target site. In a preferred embodiment the sequence of the first homology arm is at least 99 % identical to the genomic sequence upstream of the at least one target site and the sequence of the second homology arm is at least 99 % identical to the genomic sequence downstream of the at least one target site. In a preferred embodiment the sequence of the first homology arm is 100 % identical to the genomic sequence upstream of the at least one target site and the sequence of the second homology arm is 100 % identical to the genomic sequence downstream of the at least one target site.

In a preferred embodiment the sequence of the first homology arm is at least 81, 82, 83, 84, 85, 86m 87, 88, 89, 90, 91, 92, 93, 94, 95, 96, 97, 98 or 99 % identical to the genomic sequence upstream of the at least one target site.

In a preferred embodiment the sequence of the second homology arm is at least 81, 82, 83, 84, 85, 86m 87, 88, 89, 90, 91, 92, 93, 94, 95, 96, 97, 98 or 99 % identical to the genomic sequence downstream of the at least one target site.

There can be at least one target site, i.e. there can be more than one target sites. In a preferred embodiment there is one target site.

In a preferred embodiment the sequence of the first homology arm is at least 80 % identical to the genomic sequence upstream of the target site and the sequence of the second homology arm is at least 80 % identical to the genomic sequence downstream of the target site.

In a preferred embodiment the sequence of the first homology arm is at least 81, 82, 83, 84, 85, 86m 87, 88, 89, 90, 91, 92, 93, 94, 95, 96, 97, 98 or 99 % identical to the genomic sequence upstream of the target site.

In a preferred embodiment the sequence of the second homology arm is at least 81, 82, 83, 84, 85, 86m 87, 88, 89, 90, 91, 92, 93, 94, 95, 96, 97, 98 or 99 % identical to the genomic sequence downstream of the target site.

In an alternative embodiment there can be also 2 target sites, 3 target sites, 4 target sites, 5 target sites or more than 5 target sites.

The method according to the present invention can be used for example also in genome editing approaches that require or benefit from the absence of off-target effects and/or are suffering from low editing efficiencies, e.g. in case of knock-in or complex knock-out edits and/or do not allow the direct selection of edited cells based on phenotypes and/or require or benefit from the automatic removal of unedited and wrongly edited cells, leading to pure populations consisting of target edited cells.

The present invention refers also to a kit comprising an endonuclease with ability of collateral cleavage of RNA and/or DNA and specific gRNA and/or a donor DNA. Preferably, the endonuclease is an endonuclease as described herein.

Preferably the kit according to the present invention is for use in a method according to the present invention.

Preferably, the kit comprises a manual for use of the kit in a method according to the present invention.

The present invention refers also to a use of an endonuclease with ability of collateral cleavage of RNA and/or DNA for editing the genomic sequence of at least one cell at a specific sequence site. Preferably, the endonuclease is an endonuclease as described herein.

Preferred embodiments of the invention are also disclosed in the dependent claims.

The invention will be explained in more detail in the examples and the figures.
- Fig. 1: shows the mode of action of CA nucleases.
- Fig. 2: shows schematically the method according to the present invention.

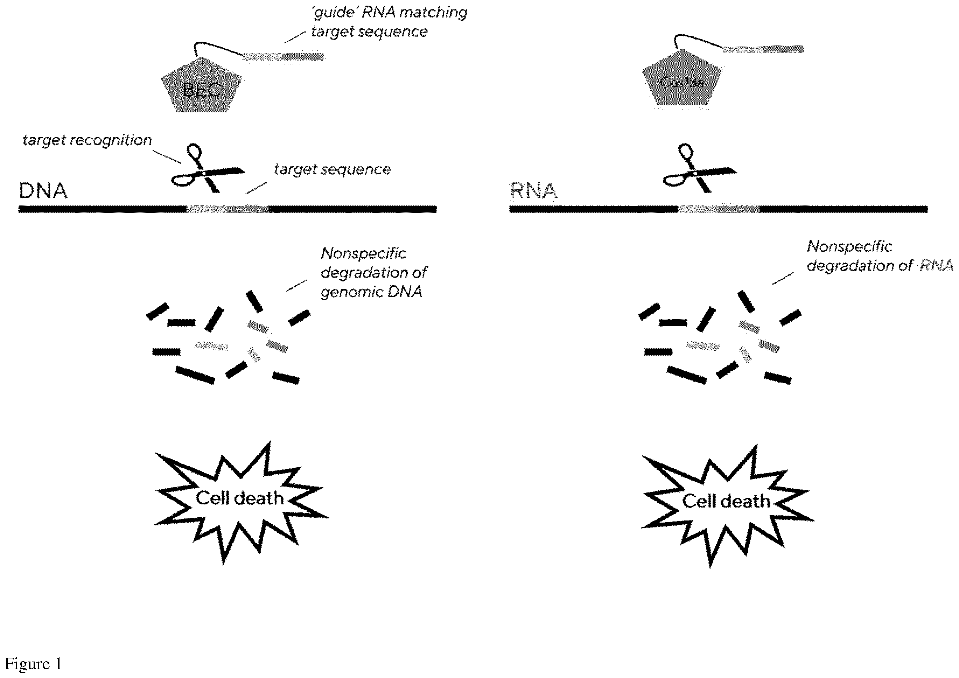

Figure 1 shows schematically the mode of action of CA nucleases. A so-called guide RNA (gRNA) that is characterized by a sequence complementary to a sequence stretch of the targeted RNA/genomic DNA is designed. The CA nuclease and the specific gRNA are transferred to or produced in the cell. The gRNA binds to the CA nuclease and guides it to the target sequence on the RNA/genomic DNA. Target recognition triggers the collateral activity of the nuclease resulting in the nonspecific degradation of RNA/genomic DNA. The cell dies due to the extensive, universal deletion of RNA/DNA.

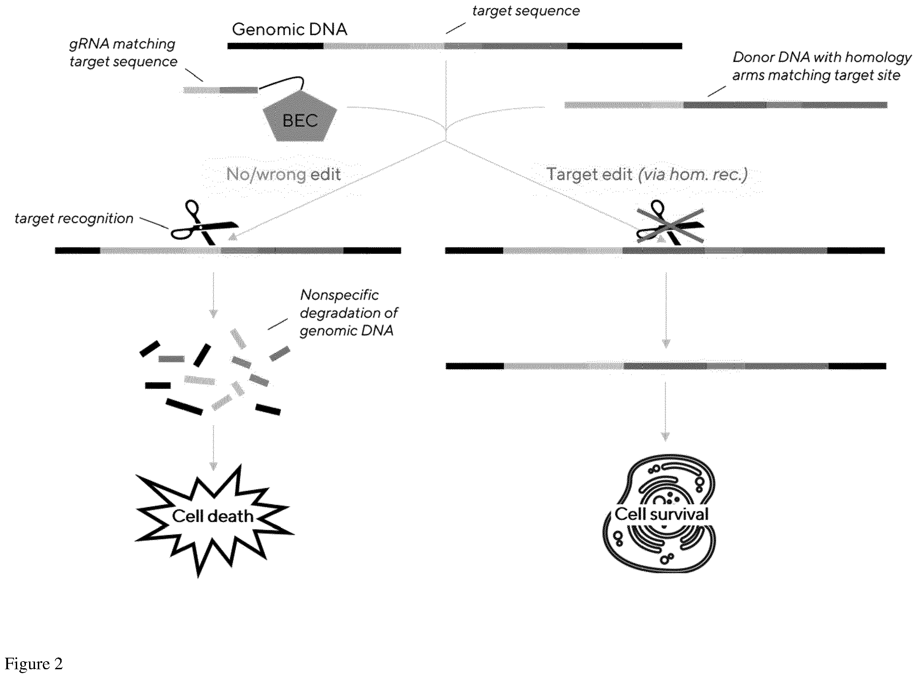

Figure 2 shows schematically the method according to the present invention, wherein a donor DNA is created that contains the desired edited sequence flanked by an upstream and a downstream sequence (= homology arms) that are identical with the upstream/downstream sequences present around the target site in the genome. Then, a functional gRNA that has a sequence overlapping with the target site sequence in the genome is designed. The donor DNA, gRNA and CA nuclease, in the present case BEC, are made available in the target cells. There are a number of options, in which form the individual components can be delivered to the cells. Furthermore, the delivery of the components can take place in a combined or stepwise fashion. Upon delivery of the donor DNA, editing of the target site takes place by natural homologous recombination. The frequency of these editing events strongly depends on the origin of the targeted cells, but is typically relatively low (e.g., for mammalian cells the frequency is usually < 0.01 %). In all non-edited cells, target recognition takes place resulting in nonspecific RNA/DNA degradation and cell death. In all edited cells, target recognition does not occur, the CA nuclease is not activated and the cells survive. Based on this mechanism, unedited and wrongly edited cells are automatically removed and only the desired, correctly edited cells remain.

The described approach allows for genome editing without off-target effects as well as for concomitant removal of unedited and wrongly edited cells, leading to enriched or essentially pure populations consisting of target edited cells.

### EXAMPLES

### Example 1: Knock-out of FUT8 activity in CHO cells and enrichment of correctly edited cells using BEC10 nuclease

The goal of the present experiment is the generation and selection of engineered CHO cells with inactivated fucosyltransferase, by the insertion of stop codons in Exon 3 of the FUT8 gene. The donor DNA is synthesized as ssDNA fragment (SEQ ID No. 2) in which the stop codon sequence (SEQ ID No. 1) is flanked by ca. 750 bp FUT8 sequences surrounding the desired integration site as the 5' and 3' homology arms. 2E6 CHO DG44 cells are transfected with 150 pmol BEC10 nuclease, 600 pmol synthetic gRNA (SEQ ID No. 3) and 4 µg donor DNA by electroporation using a Neon system from ThermoFisher. For comparison reasons, two additional transfections are executed as follows:
- Comparison 1: 150 pmol MAD7 nuclease. 600 pmol synthetic gRNA (SEQ ID No. 4) and 4 µg donor DNA
- Comparison 2: 4 µg donor DNA

Following transfection, the cells are transferred into culture medium and cultivated. After 3 days, genomic DNA is isolated and amplified by PCR using primers flanking the target site followed by Sanger sequencing. The generated sequencing data are analyzed using the ICE analysis tool provided by Synthego (https://ice.synthego.com/#/) in order to quantify the portion of correctly edited cells and compare the three different transfection approaches with each other. measure editing efficiencies and confirm successful editing.

### Example 2: Knock-out of FUT8 activity in CHO cells and enrichment of correctly edited cells using Cas13 nuclease

The goal of the present experiment is the generation and selection of engineered CHO cells with inactivated fucosyltransferase, by the insertion of stop codons in Exon 3 of the FUT8 gene. The donor DNA is synthesized as ssDNA fragment in which the stop codon sequence (SEQ ID No. 1) is flanked by ca. 750 bp FUT8 sequences surrounding the desired integration site as the 5' and 3' homology arms. 2E6 CHO DG44 cells are transfected with Cas13 nuclease, synthetic gRNA and 4 µg donor DNA by electroporation using a Neon system from ThermoFisher. For comparison reasons, two additional transfections are executed as follows:
- Comparison 1: 150 pmol MAD7 nuclease. 600 pmol synthetic gRNA and 4 µg donor DNA
- Comparison 2: 4 µg donor DNA

Following transfection, the cells are transferred into culture medium and cultivated. After 3 days, genomic DNA is isolated and amplified by PCR using primers flanking the target site followed by Sanger sequencing. The generated sequencing data are analyzed using the ICE analysis tool provided by Synthego (https://ice.synthego.com/#/) in order to quantify the portion of correctly edited cells and compare the three different transfection approaches with each other.
SEQ ID No. 1:
   TGATAGGTGAATAA
SEQ ID No. 2: 
gtagtgagatgacagcgagatggagtgatgagaatttgtagaaatgaattcacttatactgagaacttgttttgcttttagataatgaacatatta
gcctgaagtacatagccgaattgattaattattcaaagatataatcttttaatccctataaaagaggtattacacaacaattcaagaaagatagaa
ttagacttccagtattggagtgaaccatttgttatcaggtagaaccctaacgtgtgtggttgacttaaagtgtttactttttacctgatactgggtag
ctaattgtctttcagcctcctggccaaagataccatgaaagtcaacttacgttgtattctatatctcaaacaactcagggtgtttcttactctttcca
cagcatgtagagcccaggaagcacaggacaagaaagctgcctccttgtatcaccaggaagatctttttgtaagagtcatcacagtataccag
agagactaattttgtctgaagcatcatgtgttgaaacaacagaaacttattttcctgtgtggctaactagaaccagagtacaatgtttccaattcttt
gagctccgagaagacagaagggagttgaaactctgaaaatgcgggcatggactggttcctggcgttggattatgctcattctttttgcctggg
ggaccttattgttttatataggtggtcatttggttcgagataatgaccaccctgaccattctagcagagaaCTCTCC
TGATAGGTGAATAACAACAAAAT
gaagacttgaggagaatggctgagtctctccggtaggtttgaaatactcaaggatttgatgaaatactgtgcttgacctttaggtatagggtctc
agtctgctgttgaaaaatataatttctacaaaccgtctttgtaaaattttaagtattgtagcagactttttaaaagtcagtgatacatctatatagtca
atataggtttacatagttgcaatcttattttgcatatgaatcagtatatagaagcagtggcatttatatgcttatgttgcatttacaattatgtttagac
gaacacaaactttatgtgatttggattagtgctcattaaatttttttattctatggactacaacagagacataaattttgaaaggcttagttactcttaa
attcttatgatgaaaagcaaaaattcattgttaaatagaacagtgcatctggaatgtgggtaattattgccatatttctagtctactaaaaattgtgg
cataactgttcaaagtcatcagttgtttggaaagccaaagtctgatttaaatggaaaacataaacaatgatatctatttctagatacctttaacttgc
agttactgagtttacaagttgtctgacaactttggattctcttacttcatatctaagaatgatcatgtgtacagtgcttactgtcactttaaaaaactg
cagggctagacatgcagatatgaa
SEQ ID No. 3:
   AAUUUCUACUGUUGUAGAUAGCGCUCCAGCUUUGCAAGAA
SEQ ID No. 4:

GUCAAAAGACCUUUUUAAUUUCUACUCUUGUAGAUAGCGCUCCAGCUUUGCAAG
AA

## Claims

1. A method for editing the genomic sequence of at least one cell at at least one specific target site comprising the steps:
a) introducing into the at least one cell an endonuclease with ability of collateral cleavage of RNA and/or DNA or a DNA or RNA with a sequence encoding such an endonuclease, at least one donor DNA comprising the at least one edited sequence of the at least one specific target site and at least one guide RNA (gRNA) or a DNA coding for such a gRNA, and
b) cultivating the at least one cell,
wherein the at least one guide RNA comprises a sequence binding to the genomic sequence of the at least one target site,
wherein the at least one donor DNA comprises a first homology arm located upstream of the edited sequence and a second homology arm located downstream of the edited sequence, wherein the sequence of the first homology arm is at least 80 % identical to the genomic sequence upstream of the at least one target site and the sequence of the second homology arm is at least 80 % identical to the genomic sequence downstream of the at least one target site.

2. The method according to claim 1, wherein a culture of one cell or of at least 100 cells, more preferably of at least 10000 cells, even more preferably 1000000 cells is used.

3. The method according to any of the preceding claims, wherein the at least one cell or the cells of the cell culture are mammalian cells.

4. The method according to any of the preceding claims, comprising the further step
c) isolating the living cells.

5. The method according to any of the preceding claims, wherein in step a1) the at least one donor DNA is introduced into the at least one cell as linear ssDNA and wherein in following step a2) the at least one guide RNA is introduced into the at least one cell as synthetic RNA together with the endonuclease protein.

6. The method according to any of the preceding claims, wherein the endonuclease or a nucleotide with a sequence encoding the endonuclease, the at least one donor DNA comprising the edited sequence and the at least one guide RNA are introduced into the at least one cell via transfection.

7. The method according to any of the preceding claims, wherein the endonuclease or a DNA or RNA with a sequence encoding the endonuclease, the at least one donor DNA comprising the edited sequence and the at least one guide RNA or a DNA coding for such a gRNA are introduced into the at least one cell together in one transfection step or in sequential separate steps in any order and combination.

8. The method according to any of the preceding claims, wherein the endonuclease is a CRISPR-associated endonuclease.

9. The method according to any of the preceding claims, wherein the endonuclease comprises collateral RNA cleavage activity, preferably is a type III or VI Cas nuclease, more preferably a type VI Cas nuclease, more preferably a Cas13 nuclease, most preferably a Cas13a nuclease.

10. The method according to any of claims 1 to 9, wherein the endonuclease comprises collateral DNA cleavage activity, preferably is a BEC nuclease.

11. Kit comprising an endonuclease with ability of collateral cleavage of RNA and/or DNA and specific gRNA and/or a donor DNA.

12. Kit according to claim 11 for use in a method according to claims 1 to 10.

13. Kit according to claims 11 and 12, comprising a manual for use of the kit in a method according to claims 1 to 10.

14. Use of an endonuclease with ability of collateral cleavage of RNA and/or DNA for editing the genomic sequence of at least one cell at a specific sequence site.
